(19) **Europäisches Patentamt / European Patent Office / Office européen des brevets**

(11) **EP 3 971 202 B1**

(12) **EUROPEAN PATENT SPECIFICATION**

(45) Date of publication and mention
of the grant of the patent:
**31.12.2025  Bulletin 2026/01**

(21) Application number: **20805342.1**

(22) Date of filing: **15.05.2020**

(51) International Patent Classification (IPC):
*C12N 5/00* (2006.01)     *C12M 1/00* (2006.01)
*C07K 5/11* (2006.01)     *C07K 7/06* (2006.01)
*C08F 8/30* (2006.01)     *C08F 261/12* (2006.01)

(52) Cooperative Patent Classification (CPC):
(C-Sets available)
**C12N 5/0068; C08F 8/30; C08F 261/12;
C12M 23/20;** C07K 5/1019; C07K 7/06;
C12N 2533/30; C12N 2533/50     (Cont.)

(86) International application number:
**PCT/JP2020/019416**

(87) International publication number:
**WO 2020/230886 (19.11.2020 Gazette 2020/47)**

(54) **RESIN FILM FORMED OF SCAFFOLD MATERIAL FOR CELL CULTURE, CARRIER FOR CELL CULTURE AND CONTAINER FOR CELL CULTURE**

AUS GERÜSTMATERIAL GEFORMTER HARZFILM ZUR ZELLZÜCHTUNG, TRÄGER FÜR ZELLZÜCHTUNG UND BEHÄLTER FÜR ZELLZÜCHTUNG

FILM DE RÉSINE FORMÉ D'UN MATÉRIAU D'ÉCHAFAUDAGE POUR CULTURE CELLULAIRE, SUPPORT DE CULTURE CELLULAIRE ET RÉCIPIENT DE CULTURE CELLULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.05.2019   JP 2019092083
26.06.2019   JP 2019119079**

(43) Date of publication of application:
**23.03.2022   Bulletin 2022/12**

(73) Proprietor: **SEKISUI CHEMICAL CO., LTD.
Osaka-shi
Osaka
530-8565 (JP)**

(72) Inventors:
• **IGUCHI, Hiroki
Mishima-gun, Osaka 618-0021 (JP)**
• **ARAI, Yuuhei
Mishima-gun, Osaka 618-0021 (JP)**
• **INUI, Nobuhiko
Hasuda-shi, Saitama 349-0198 (JP)**
• **YUKAWA, Mayumi
Mishima-gun, Osaka 618-0021 (JP)**
• **KOBAYASHI, Daigo
Mishima-gun, Osaka 618-0021 (JP)**
• **NAKAMURA, Yuuta
Mishima-gun, Osaka 618-0021 (JP)**
• **TAKAKURA, Kenta
Mishima-gun, Osaka 618-0021 (JP)**
• **HANEDA, Satoshi
Mishima-gun, Osaka 618-0021 (JP)**
• **ISHII, Ryoma
Tokyo 105-8566 (JP)**

(74) Representative: **Ter Meer Steinmeister & Partner
Patentanwälte mbB
Nymphenburger Straße 4
80335 München (DE)**

(56) References cited:
| | |
|---|---|
| EP-A1- 3 733 834 | EP-A1- 3 733 835 |
| WO-A1-2018/181758 | WO-A1-2019/131978 |
| WO-A1-2019/131982 | WO-A2-2007/070660 |
| JP-A- 2001 089 574 | JP-A- 2006 314 285 |
| JP-A- 2009 519 042 | JP-A- 2019 118 345 |

EP 3 971 202 B1

- **HIRAGUCHI YUKARI: "Control of adsorbed proteins by nanoscale phase-separated polymer structures and subsequent induction of cell adhesion", THESIS OF THE UNIVERSITY OF TOKYO, 22 March 2018 (2018-03-22), pages 1 - 5, XP055760546**

Remarks:
The complete document including Reference Table(s) and the Sequence Listing(s) can be downloaded from the EPO website

(52) Cooperative Patent Classification (CPC): (Cont.)

C-Sets
**C08F 8/30, C08F 261/12;**
**C08F 261/12, C08F 220/06**

## Description

### TECHNICAL FIELD

The present invention relates to a resin film formed of a cell culture scaffold material. The present invention also relates to a cell culture carrier and a cell culture vessel including the resin film.

### BACKGROUND ART

In recent years, next-generation medicine using cell medicine or stem cells has been attracting attention. Among them, human pluripotent stem cells (hPSC) such as human embryonic stem cells (hESC) or human induced pluripotent stem cells (hiPSC) or differentiated cells derived from them are expected to be applied to drug discovery and regenerative medicine. In order to achieve such an application, it is necessary to culture and proliferate pluripotent stem cells and differentiated cells safely and with good reproducibility.

In particular, for industrial use in regenerative medicine, it is necessary to handle a large amount of stem cells, so it becomes necessary to support proliferation of pluripotent stem cells using natural polymer materials, synthetic polymer materials, or feeder cells. Therefore, various culture methods using scaffolding materials such as natural polymer materials and synthetic polymer materials have been studied.

For example, Patent Document 1 below discloses a cell culture carrier composed of a molded product made of a polyvinyl acetal compound or a molded product made of the polyvinyl acetal compound and a water-soluble polysaccharide, in which the polyvinyl acetal compound has a degree of acetalization of 20 to 60 mol%.

Patent Document 2 below discloses a composition containing a first fiber polymer scaffolding, in which the fibers of the first fiber polymer scaffolding are aligned. It is described that the fibers constituting the first fiber polymer scaffolding are composed of an aliphatic polyester such as polyglycolic acid or polylactic acid.

Further, Patent Document 3 below describes a cell culture method for maintaining undifferentiation of pluripotent stem cells, including a step of culturing the pluripotent stem cells on an incubator having a surface coated with a polyrotaxan block copolymer.

### Related Art Document

### Patent Document

    Patent Document 1: JP 2006-314285 A
    Patent Document 2: JP 2009-524507 W
    Patent Document 3: JP 2017-23008 A

### SUMMARY OF THE INVENTION

### PROBLEMS TO BE SOLVED BY THE INVENTION

Incidentally, when a natural polymer material is used as a scaffold material, fixation of cells after seeding can be enhanced. In particular, it is known that the fixation of cells after seeding is extremely high when an adhesive protein such as laminin or vitronectin, or a matrigel derived from mouse sarcoma is used as a natural polymer. On the other hand, natural polymer materials are expensive, have large variations between lots because they are naturally derived substances, or have safety concerns due to animal-derived components.

In contrast, the scaffold materials using synthetic resins have good operability, are inexpensive, have less variation between lots and are excellent in safety, in comparison to scaffold materials using natural polymer materials. However, in the scaffolding materials using synthetic resins as in Patent Documents 1 to 3, the synthetic resins swell excessively because synthetic resins having high hydrophilicity are used. In addition, since synthetic resins have lower affinity for cells than natural polymer materials, a cell mass may exfoliate during culturing. As described above, the scaffolding materials using synthetic resins have low fixation of cells after seeding, and the cells may not proliferate sufficiently.

An object of the present invention is to provide a resin film formed of a cell culture scaffold material, a cell culture carrier, and a cell culture vessel, which have excellent fixation of cells after seeding and are capable of enhancing proliferation rate of cells.

## MEANS FOR SOLVING THE PROBLEMS

In a resin film formed of a cell culture scaffold material according to the present invention, the cell culture scaffold material contains a synthetic polyvinyl acetal resin having a polyvinyl acetal resin portion, a linker portion, and a peptide portion, the resin film has a phase-separated structure including at least a first phase and a second phase, and a ratio of the surface area of one of the first phase and the second phase to the entire surface is 0.01 or more and 0.95 or less.

In a specific aspect of the resin film according to the present invention, the ratio of the peripheral length to the area of the second phase (peripheral length/area) is 0.001 (1/nm) or more and 0.40 (1/nm) or less.

In another specific aspect of the resin film according to the present invention, the phase-separated structure is a sea-island structure, the first phase is a sea part, and the second phase is an island part.

In still another specific aspect of the resin film according to the present invention, the number of the second phase as an island part is 1 piece/$\mu m^2$ or more and 5,000 pieces/$\mu m^2$ or less.

In still another specific aspect of the resin film according to the present invention, the phase-separated structure is composed of a phase-separated structure within a molecule of the synthetic resin.

In still another specific aspect of the resin film according to the present invention, the synthetic resin has a cationic functional group, and the content of the cationic functional group contained in a structural unit of the synthetic resin is 0.2 mol% or more and 50 mol% or less.

In still another specific aspect of the resin film according to the present invention, the second phase has a peptide portion.

In still another specific aspect of the resin film according to the present invention, the peptide portion has a cell-adhesive amino acid sequence.

In still another specific aspect of the resin film according to the present invention, the resin film has a water swelling ratio of 50% or less.

In still another specific aspect of the resin film according to the present invention, the resin film has a storage elastic modulus at 100°C of $1.0 \times 10^4$ Pa or more and $1.0 \times 10^8$ Pa or less, and a ratio of a storage elastic modulus at 25°C to a storage elastic modulus at 100°C ((storage elastic modulus at 25°C)/(storage elastic modulus at 100°C)) of $1.0 \times 10^1$ or more and $1.0 \times 10^5$ or less.

In still another specific aspect of the resin film according to the present invention, the cell culture scaffold material does not substantially contain animal-derived raw materials.

In still another specific aspect of the resin film according to the present invention, the synthetic resin contains a vinyl polymer.

In still another specific aspect of the resin film according to the present invention, the synthetic resin contains at least a polyvinyl alcohol derivative or a poly(meth)acrylic acid ester.

The cell culture carrier according to the present invention includes a carrier, and a resin film constituted according to the present invention, and the resin film is arranged on a surface of the carrier.

The cell culture vessel according to the present invention includes a vessel body, and a resin film constituted according to the present invention, and the resin film is arranged on a surface of the vessel body.

## EFFECT OF THE INVENTION

According to the present invention, it is possible to provide a resin film formed of a cell culture scaffold material, a cell culture carrier, and a cell culture vessel, which have excellent fixation of cells after seeding and are capable of enhancing proliferation rate of cells.

## BRIEF DESCRIPTION OF DRAWINGS

[Fig. 1] Fig. 1 is a schematic front cross-sectional view showing a cell culture vessel according to an embodiment of the present invention.
[Fig. 2] Fig. 2 is an atomic force micrograph of a resin film obtained in Example 3.

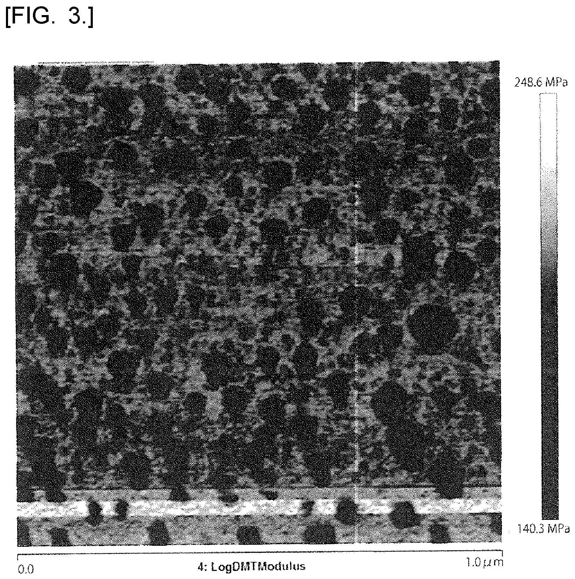

[Fig. 3] Fig. 3 is an atomic force micrograph of a resin film obtained in Example 14.

## MODES FOR CARRYING OUT THE INVENTION

Hereinafter, the present invention will be clarified by explaining specific embodiments of the present invention with reference to the drawings.

The present invention relates to a resin film formed of a cell culture scaffold material. The cell culture scaffold material contains a synthetic resin. The resin film of the present invention has a phase-separated structure including at least a first phase and a second phase. In the resin film of the present invention, a ratio of the surface area of one of the first phase and the second phase to the entire surface is 0.01 or more and 0.95 or less.

Since the resin film of the present invention has the above structure, it has excellent fixation of cells after seeding and is capable of enhancing proliferation rate of cells.

Conventional cell culture scaffold materials using natural polymer materials can enhance fixation of cells after seeding, but are expensive, have large variations between lots because they are naturally derived substances, or have safety concerns due to animal-derived components. On the other hand, in the conventional scaffolding materials using synthetic resins, the synthetic resins swell excessively or have low affinity for cells, so that a cell mass may exfoliate during culturing. Therefore, the conventional scaffolding materials using synthetic resins have low fixation of cells after seeding, and the cells may not proliferate sufficiently.

The present inventors have focused on a phase-separated structure of a resin film formed of a cell culture scaffold material, and have found that a phase-separated structure having a ratio of the surface area of one of the first phase and the second phase to the entire surface in the above specific range can enhance the affinity for cells, thereby capable of enhancing adhesion after seeding, and thus enhancing the proliferation rate of cells. A reason for this is not clear, but when having such a phase-separated structure, energy distribution proceeds smoothly, and positions and ratios of the first phase and the second phase having different affinities and intensities can be adjusted. Therefore, it is considered that the affinity can be enhanced regardless of the type of cell, and accumulation and adsorption effect of cells or cell surface proteins can be realized.

Therefore, according to the resin film of the present invention, adhesiveness to the cells after seeding can be enhanced, and the proliferation rate of cells can be enhanced.

Further, in the present invention, since the synthetic resin can be used as described above, it has good operability, is inexpensive, has less variation between lots and is excellent in safety, in comparison to scaffold materials using natural polymer materials.

In the present invention, a synthetic resin having a peptide portion may be used as the synthetic resin. Details of the synthetic resin having a peptide portion will be described later.

In the present invention, the ratio of the surface area of one of the first phase and the second phase to the entire surface (surface area fraction) is 0.01 or more, preferably 0.10 or more, 0.95 or less, and more preferably 0.90 or less. When the surface area fraction is within the above range, the adhesiveness to the cells after seeding can be further enhanced, and the proliferation rate of cells can be further enhanced.

In the present invention, examples of the phase-separated structure include microphase-separated structures such as a sea-island structure, a cylinder structure, a gyroid structure, or a lamellar structure. In the sea-island structure, for example, the first phase can be a sea part and the second phase can be an island part. In the cylinder structure, gyroid structure, or lamellar structure, for example, a phase having a largest surface area can be the first phase, and a phase having a second largest surface area can be the second phase. Among these, the sea-island structure is preferable as the phase-separated structure. As described above, by having a continuous phase and a discontinuous phase, it is possible to enhance the affinity for cells and further enhance the adhesiveness to the cells after seeding, thereby further enhancing the proliferation rate of cells.

When the phase-separated structure is a sea-island structure, the surface area fraction of the second phase to the entire surface is 0.01 or more, preferably 0.1 or more, and more preferably 0.2 or more, and 0.95 or less, preferably 0.9 or less, and more preferably 0.8 or less. When the surface area fraction is within the above range, the adhesiveness to the cells after seeding can be further enhanced, and the proliferation rate of cells can be further enhanced.

A ratio of the peripheral length to the area of the second phase (peripheral length/area) is preferably 0.001 (1/nm) or more, more preferably 0.0015 (1/nm) or more, and further preferably 0.008 (1/nm) or more. The ratio of the peripheral length to the area of the second phase (peripheral length/area) is preferably 0.40 (1/nm) or less, more preferably 0.20 (1/nm) or less, further preferably 0.08 (1/nm) or less, and particularly preferably 0.013 (1/nm) or less. When the ratio (peripheral length/area) is within the above range, the adhesiveness to the cells after seeding can be further enhanced, and the proliferation rate of cells can be further enhanced.

When the synthetic resin does not have a peptide portion, the ratio of the peripheral length to the area of the second phase (peripheral length/area) is preferably 0.001 (1/nm) or more, more preferably 0.0015 (1/nm) or more, preferably 0.08 (1/nm) or less, and more preferably 0.013 (1/nm) or less. When the ratio (peripheral length/area) is within the above range, the adhesiveness to the cells after seeding can be further enhanced, and the proliferation rate of cells can be further enhanced.

When the synthetic resin has a peptide portion, the ratio of the peripheral length to the area of the second phase (peripheral length/area) is preferably 0.008 (1/nm) or more, more preferably 0.013 (1/nm) or more, preferably 0.40 (1/nm) or less, more preferably 0.20 (1/nm) or less, and further preferably 0.10 (1/nm) or less. When the ratio (peripheral length/area) is within the above range, the adhesiveness to the cells after seeding can be further enhanced, and the proliferation rate of cells can be further enhanced.

The number of the second phases as island parts is preferably 1 piece/$\mu$m$^2$ or more, more preferably 2 pieces/$\mu$m$^2$ or more, further preferably 10 pieces/$\mu$m$^2$ or more, preferably 5,000 pieces/$\mu$m$^2$ or less, more preferably 1,000 pieces/$\mu$m$^2$ or less, further preferably 500 pieces/$\mu$m$^2$ or less, and particularly preferably 300 pieces/$\mu$m$^2$ or less. In this case, the adhesiveness to the cells after seeding can be further enhanced, and the proliferation rate of cells can be further enhanced.

The average diameter of the second phases as island parts is preferably 20 nm or more, more preferably 30 nm or more, further preferably 50 nm or more, particularly preferably 80 nm or more, preferably 3.5 $\mu$m or less, more preferably 3.0 $\mu$m or less, and further preferably 1.5 $\mu$m or less. When the average diameter of the second phases is within the above range, the adhesiveness to the cells after seeding can be further enhanced, and the proliferation rate of cells can be further enhanced.

When the synthetic resin does not have a peptide portion, the average diameter of the second phases as island parts is preferably 50 nm or more, more preferably 100 nm or more, further preferably 120 nm or more, particularly preferably 200 nm or more, preferably 1 $\mu$m or less, more preferably 300 nm or less, and further preferably 250 nm or less. When the average diameter of the second phases is within the above range, the adhesiveness to the cells after seeding can be further enhanced, and the proliferation rate of cells can be further enhanced.

When the synthetic resin has a peptide portion, the average diameter of the second phases as island parts is preferably 10 nm or more, more preferably 20 nm or more, further preferably 40 nm or more, preferably 1 $\mu$m or less, more preferably 300 nm, and further preferably 100 nm or less. When the average diameter of the second phases is within the above range, the adhesiveness to the cells after seeding can be further enhanced, and the proliferation rate of cells can be further enhanced.

The presence or absence of a phase-separated structure and parameters indicating the phase-separated structure as described above can be confirmed by, for example, an atomic force microscope (AFM), a transmission electron microscope (TEM), a scanning electron microscope (SEM), or the like.

Specifically, the ratio of the surface area of one of the first phase and the second phase to the entire surface (surface area fraction), a ratio of the peripheral length to the area of the second phase (peripheral length/area), the number of the second phases as island parts and the average diameter size thereof can be obtained from the above-mentioned microscopic observation image using image analysis software such as ImageJ.

The ratio of the surface area of one of the first phase and the second phase to the entire surface (surface area fraction) is obtained by, within an observation region (30 $\mu$m $\times$ 30 $\mu$m), dividing a surface area occupied by one of the first phase and the second phase by an area of the observation region.

The ratio of the peripheral length to the area of the second phase (peripheral length/area) is obtained by, within the observation region (30 $\mu$m $\times$ 30 $\mu$m), dividing a total peripheral length of the second phase by a total area of the second phase.

When the phase-separated structure is a sea-island structure, the number of second phases as island parts can be obtained by dividing the number of second phases in the observation region (30 $\mu$m $\times$ 30 $\mu$m) by the area of the observation region. Also, the average diameter of the second phases as island parts is obtained as an average diameter of circles of the same area.

Further, the phase-separated structure as described above can be obtained by, for example, blending at least two different types of polymers, copolymerizing, graft-copolymerizing, or using a synthetic resin having a peptide portion to form a phase-separated structure between molecules or within a molecule of the synthetic resin. Among them, from the viewpoint of further enhancing cell adhesion, it is preferable that the phase-separated structure is formed of a phase-separated structure within a molecule. That is, the synthetic resin is preferably a copolymer of at least two different types of polymers or a synthetic resin having a peptide portion, and is more preferably a graft copolymer or a synthetic resin having a peptide portion.

The phase-separated structure as described above is preferably obtained by copolymerizing two or more different types of polymers (monomers) having a solubility parameter (SP value) of 0.1 or more, preferably 0.5 or more, and more preferably 1 or more. In this case, the sea-island structure can be formed more easily.

The SP value is a measure of intermolecular force acting between a solvent and a solute, and is a measure of affinity between substances. The SP value can be determined based on Hidebrand's theory of regular solutions. In addition to being able to obtain the SP value from literature information, it can also be obtained by calculation method of Hansen and Hoy, Fedors' estimation method, and the like. In this specification, it means a calculated value calculated by Fedors' equation $\delta^2 = \Sigma E/\Sigma V$ ($\delta$ means SP value, E means evaporation energy, and V means molar volume). A unit of SP value is (cal/cm$^3$)$^{0.5}$. The Fedors method is described in Journal of the Adhesion Society of Japan, 1986, Vol. 22, p. 566.

The phase separation parameters indicating the phase-separated structure such as the surface area fraction can be adjusted by, for example, controlling a blending ratio of the two types of polymers or structure of the polymers, or controlling the content of the peptide portion.

In the present invention, there may be other phase different from the first phase and the second phase. The other phase may be one phase or a plurality of phases. Such a phase can be obtained, for example, by copolymerization such as grafting still another polymer (monomer) having a different SP value. In this case, two phases occupying large areas of the surface of the resin film are defined as the first phase and the second phase.

When the synthetic resin does not have a peptide portion, a dispersion term component of surface free energy in the resin film formed of the cell culture scaffold material is preferably 25.0 mJ/m$^2$ or more and 50.0 mJ/m$^2$ or less. In this case, hydrophilicity of the cell culture scaffold material can be appropriately adjusted, and by a synergistic effect with the phase-separated structure, interfacial adhesiveness to the cells after seeding can be further enhanced, and the proliferation rate of cells can be further enhanced. The dispersion term component is more preferably 30.0 mJ/m$^2$ or more, further preferably 35.0 mJ/m$^2$ or more, more preferably 47.0 mJ/m$^2$ or less, and further preferably 45.5 mJ/m$^2$ or less.

When the synthetic resin does not have a peptide portion, a polar term component of surface free energy in the resin film formed of the cell culture scaffold material is preferably 1.0 mJ/m$^2$ or more and 20.0 mJ/m$^2$ or less. In this case, the adhesiveness to the cells after seeding can be further enhanced, and the proliferation rate of cells can be further enhanced. The polar term component is more preferably 2.0 mJ/m$^2$ or more, further preferably 3.0 mJ/m$^2$ or more, more preferably 10.0 mJ/m$^2$ or less, and further preferably 5.0 mJ/m$^2$ or less.

Dispersion term component $\gamma^d$ of surface free energy and dipole component $\gamma^p$ as a polar term component of surface free energy are calculated using the Kaelble-Uy theoretical formula. The Kaelble-Uy theoretical formula is a theoretical formula based on an assumption total surface free energy $\gamma$ is a sum of the dispersion term component $\gamma^d$ and the dipole component $\gamma^p$, as shown by Formula (1) below.
[Expression 1]

$$\gamma = \gamma^d + \gamma^p \qquad (1)$$

In the Kaelble-Uy's theoretical formula, when surface free energy of liquid is $\gamma_l$ (mJ/m$^2$), surface free energy of solid is $\gamma_s$ (mJ/m$^2$), and contact angle is $\theta$ (°), the Formula (2) below is established.
[Expression 2]

$$\gamma_l (1 + \cos\theta) = 2\sqrt{\gamma_s^d \gamma_l^d} + 2\sqrt{\gamma_s^p \gamma_l^p} \qquad (2)$$

Therefore, contact angle $\theta$ with respect to the resin film formed of the cell culture scaffold material is measured using two types of liquids whose surface free energy of liquid $\gamma_l$ is known, and simultaneous equations of $\gamma_s^d$ and $\gamma_s^p$ are solved, whereby the dispersion term component $\gamma^d$ and dipole component $\gamma^p$ of surface free energy of the resin film formed of the cell culture scaffold material can be obtained.

In this specification, pure water and diiodomethane are used as the two types of liquids whose surface free energy $\gamma_l$ is known.

The contact angle $\theta$ is measured as follows using a contact angle meter (for example, "DMo-701" manufactured by Kyowa Interface Science Co., Ltd.).

Pure water or diiodomethane (1 $\mu$L) is added dropwise to a surface of the resin film formed of the cell culture scaffold material. An angle formed by the pure water 30 seconds after dropping and the resin film is defined as contact angle $\theta$ with respect to the pure water. Similarly, an angle formed by the diiodomethane 30 seconds after dropping and the resin film is defined as contact angle $\theta$ with respect to the diiodomethane.

By increasing the content of hydrophobic functional groups, increasing the content of functional groups having a cyclic structure or decreasing the content of butyl groups in the synthetic resin, the dispersion term component $\gamma^d$ of the surface free energy can be reduced. Further, by increasing the content of hydrophilic functional groups or increasing the content of butyl groups in the synthetic resin, the dipole component $\gamma^p$ of the surface free energy can be reduced.

In the resin film formed of the cell culture scaffold material of the present invention, a storage elastic modulus at 100°C is preferably $0.6 \times 10^4$ Pa or more, more preferably $0.8 \times 10^4$ Pa or more, further preferably $1.0 \times 10^4$ Pa or more, preferably $1.0 \times 10^8$ Pa or less, more preferably $0.8 \times 10^8$ Pa or less, and further preferably $1.0 \times 10^7$ Pa or less.

In particular, the resin film formed from the cell culture scaffold material of the present invention has a ratio of a storage elastic modulus at 25°C to a storage elastic modulus at 100°C ((storage elastic modulus at 25°C)/(storage elastic modulus at 100°C)) is preferably $1.0 \times 10^1$ or more, more preferably $5.0 \times 10^1$ or more, further preferably $8.0 \times 10^2$ or more, preferably $1.0 \times 10^5$ or less, more preferably $0.75 \times 10^5$ or less, and further preferably $0.5 \times 10^5$ or less. By setting the storage elastic modulus within the above range, fixation of cells after seeding can be further enhanced.

The storage elastic moduli at 25°C and 100°C are measured, for example, by a dynamic viscoelasticity measuring device (manufactured by IT Keisoku Seigyo Co., Ltd., DVA-200), under tensile conditions at a frequency of 10 Hz and a temperature range of -150°C to 150°C at a heating rate of 5°C/min. The storage elastic moduli at 25°C and 100°C are obtained from a graph of the obtained tensile storage elastic modulus, and 25°C storage elastic modulus/100°C storage elastic modulus is calculated. The measurement is performed using a measurement sample with a length of 50 mm, a width of 5 to 20 mm, and a thickness of 0.1 to 1.0 mm, under conditions of 10 Hz, a strain of 0.1%, a temperature of -150°C to 150°C, and a heating rate of 5°C/min.

The storage elastic moduli at 25°C and 100°C can be increased, for example, by increasing the degree of crosslinking in the synthetic resin, stretching the synthetic resin, and the like. Further, the storage elastic moduli at 25°C and 100°C can be lowered by reducing the number average molecular weight of the synthetic resin, lowering the glass transition temperature, and the like.

The resin film formed of the cell culture scaffold material of the present invention has a water swelling ratio of preferably 50% or less and more preferably 40% or less. In this case, the fixation of cells after seeding can be further enhanced. The lower limit of the water swelling ratio is not particularly limited, but can be set to, for example, 0.5%. The water swelling ratio can be measured as follows. For example, a resin film (measurement sample) formed of a cell culture scaffold material with a length of 50 mm, a width of 10 mm, and a thickness of 0.05 mm to 0.15 mm is immersed in water at 25°C for 24 hours. Weights of the sample before and after immersion are measured, and Water swelling ratio = (Sample weight after immersion - Sample weight before immersion)/(Sample weight before immersion) $\times$ 100 (%) is calculated.

The water swelling ratio can be reduced, for example, by increasing the hydrophobic functional groups of the synthetic resin, reducing the number average molecular weight, and the like.

(Synthetic resin)

The cell culture scaffold material contains a synthetic resin (hereinafter, may be referred to as synthetic resin X). A main chain of the synthetic resin X is preferably a carbon chain. In addition, in this specification, a "structural unit" refers to a repeating unit of a monomer constituting the synthetic resin. When the synthetic resin has a graft chain, it contains a repeating unit of a monomer constituting the graft chain.

The synthetic resin X having a peptide portion may or may not have a cationic functional group in a structural part other than the peptide portion. Examples of the cationic functional group include substituents having a structure such as an amino group, an imino group, or an amide group. Examples include, without particular limitation, conjugated amine-based functional groups such as hydroxyamino group, urea group, guanidine and biguanide, heterocyclic amino functional groups such as piperazine, piperidine, pyrrolidine, 1,4-diazabicyclo[2.2.2]octane, hexamethylenetetramine, morpholine, pyridine, pyridazine, pyrimidine, pyrazine, pyrrole, azatropylidene, pyridone, imidazole, benzimidazole, benzotriazole, pyrazole, oxazole, imidazoline, triazole, thiazole, thiazine, tetrazole, indole, isoindole, purine, quinoline, isoquinoline, quinazoline, quinoxaline, cinnoline, pteridine, carbazole, acridine, adenine, guanine, cytosine, thymine, uracil and melamine, cyclic pyrrole functional groups such as porphyrin, chlorine and choline, derivatives thereof, and the like. As these cationic functional groups, one type may be used alone, or a plurality of types may be used in combination.

In the present invention, the content of the cationic functional group contained in a structural unit of the synthetic resin X is preferably 0.2 mol% or more, preferably 2 mol% or more, more preferably 3 mol% or more, 50 mol% or less, preferably 10 mol% or less, and more preferably 7 mol% or less. By using the synthetic resin X containing a cationic functional group within such a range, the fixation of cells after seeding can be further enhanced, and the proliferation rate of cells can be further enhanced. The content of the cationic functional group can be measured by, for example, $^1$H-NMR (nuclear magnetic resonance spectrum).

(Synthetic resin X having polyvinyl acetal skeleton)

The cell culture scaffold material contains synthetic resin X having a polyvinyl acetal skeleton. In the present invention, the synthetic resin X having a polyvinyl acetal skeleton is preferably a copolymer of a structural unit of a polyvinyl acetal resin and a vinyl compound and/or a vinylidene compound. A vinyl compound is a compound having a vinyl group ($H_2C=CH-$). A vinylidene compound is a compound having a vinylidene group ($H_2C=CR-$). The vinyl compound or vinylidene compound may be a vinyl polymer which is a polymer thereof. In the following description, the vinyl compound, vinylidene compound and vinyl polymer copolymerized with the polyvinyl acetal resin may be collectively referred to as "vinyl compound A".

In the present invention, the copolymer may be a block copolymer of a polyvinyl acetal resin and vinyl compound A, or may be a graft copolymer obtained by grafting vinyl compound A on a polyvinyl acetal resin. The copolymer is preferably a graft copolymer. In this case, the phase-separated structure can be formed more easily.

Examples of the vinyl compound and vinylidene compound include ethylene, allylamine, vinylpyrrolidone, maleic anhydride, maleimide, itaconic acid, (meth)acrylic acid, vinylamine, (meth)acrylic acid esters, and the like. As these vinyl compounds, only one type may be used, or two or more types may be used in combination. Therefore, it may be a vinyl polymer in which these vinyl compounds are copolymerized.

In the above copolymer, difference in SP value between the polyvinyl acetal resin and the vinyl compound A is preferably 0.5 or more. In this case, the phase-separated structure can be formed more easily. The difference in SP value between the polyvinyl acetal resin and the vinyl compound A is more preferably 1.0 or more. The upper limit of the difference in SP values is not particularly limited, but can be set to, for example, 10.0.

In the above copolymer, it is preferable that the first phase is a polyvinyl acetal resin and the second phase is vinyl compound A. It is preferable that the first phase is formed by a polyvinyl acetal resin part of the copolymer and the second phase is formed by a vinyl compound A part. In this case, it is preferable that the first phase of the polyvinyl acetal resin is a sea part and the second phase of the vinyl compound A is an island part. However, the first phase of the vinyl compound A may be a sea part, and the second phase of the polyvinyl acetal resin may be an island part.

The content fraction (mol/mol) of the vinyl compound A in the copolymer is preferably 0.015 or more, more preferably 0.3 or more, preferably 0.95 or less, more preferably 0.90 or less, and further preferably 0.70 or less. When the content fraction is the above lower limit or more, the phase-separated structure can be more easily formed. When the content fraction is the upper limit or less, the proliferation rate of cells can be further increased.

<Polyvinyl acetal resin>

Hereinafter, the polyvinyl acetal resin (polyvinyl acetal resin part of the copolymer) will be described in more detail.

The polyvinyl acetal resin has an acetal group, an acetyl group, and a hydroxyl group in side chains.

A method for synthesizing a polyvinyl acetal resin includes at least a step of acetalizing polyvinyl alcohol with an aldehyde.

The aldehyde used for acetalizing polyvinyl alcohol to obtain a polyvinyl acetal resin is not particularly limited. Examples of the aldehyde include aldehydes having 1 to 10 carbon atoms. The aldehyde may have a chain aliphatic group, a cyclic aliphatic group or an aromatic group. The aldehyde may be a chain aldehyde or a cyclic aldehyde.

Examples of the aldehyde include formaldehyde, acetaldehyde, propionaldehyde, butyraldehyde, pentanal, hexanal, heptanal, octanal, nonanal, decanal, acrolein, benzaldehyde, cinnamaldehyde, perillaldehyde, formylpyridine, formylimidazole, formylpyrrole, formylpiperidine, formyltriazole, formyltetrazole, formylindole, formylisoindole, formylpurine, formylbenzimidazole, formylbenzotriazole, formylquinoline, formylisoquinoline, formylquinoxaline, formylcinnoline, formylpteridine, formylfuran, formyloxolane, formyloxane, formylthiophene, formylthiolane, formylthiane, formyladenine, formylguanine, formylcytosine, formylthymine, formyluracil, and the like. As these aldehydes, only one type may be used, or two or more types may be used in combination.

The aldehyde is preferably formaldehyde, acetaldehyde, propionaldehyde, butyraldehyde, or pentanal, and more preferably butyraldehyde. Therefore, the polyvinyl acetal skeleton is preferably a polyvinyl butyral skeleton. The polyvinyl acetal resin is preferably a polyvinyl butyral resin.

From the viewpoint of further enhancing cell adhesion, the polyvinyl acetal resin preferably has a Bronsted basic group or a Bronsted acidic group, and more preferably has a Bronsted basic group. That is, it is preferable that a part of the polyvinyl acetal resin is modified with a Bronsted basic group or a Bronsted acidic group, and more preferably a part of the polyvinyl acetal resin is modified with a Bronsted basic group.

The Bronsted basic group is a generic term for a functional group that can receive a hydrogen ion $H^+$ from another substance. Examples of the Bronsted basic group include amine-based basic groups such as a substituent having an imine structure, a substituent having an imide structure, a substituent having an amine structure, or a substituent having an amide structure. Examples of the Bronsted basic group include, without particular limitation, conjugated amine-based functional groups such as hydroxyamino group, urea group, guanidine and biguanide, heterocyclic amino functional groups such as piperazine, piperidine, pyrrolidine, 1,4-diazabicyclo[2.2.2]octane, hexamethylenetetramine, morpholine, pyridine, pyridazine, pyrimidine, pyrazine, pyrrole, azatropylidene, pyridone, imidazole, benzimidazole, benzotriazole, pyrazole, oxazole, imidazoline, triazole, thiazole, thiazine, tetrazole, indole, isoindole, purine, quinoline, isoquinoline, quinazoline, quinoxaline, cinnoline, pteridine, carbazole, acridine, adenine, guanine, cytosine, thymine, uracil and melamine, cyclic pyrrole functional groups such as porphyrin, chlorine and choline, derivatives thereof, and the like.

Examples of the Bronsted acidic group include a carboxyl group, a sulfonic acid group, a maleic acid group, a sulfinic acid group, a sulfenic acid group, a phosphoric acid group, a phosphonic acid group, salts thereof, and the like. The Bronsted acidic group is preferably a carboxyl group.

The polyvinyl acetal resin preferably has a structural unit having an imine structure, a structural unit having an imide structure, a structural unit having an amine structure, or a structural unit having an amide structure. In this case, the polyvinyl acetal resin may have only one type of these structural units, or may have two or more types.

The polyvinyl acetal resin may have a structural unit having an imine structure. The imine structure refers to a structure having a C=N bond. In particular, the polyvinyl acetal resin preferably has an imine structure in a side chain.

The polyvinyl acetal resin may have a structural unit having an imide structure. The structural unit having an imide structure is preferably a structural unit having an imino group (=NH).

The polyvinyl acetal resin preferably has an imino group in a side chain. In this case, the imino group may be directly bonded to a carbon atom constituting a main chain of the polyvinyl acetal resin, or may be bonded to the main chain via a linking group such as an alkylene group.

The polyvinyl acetal resin may have a structural unit having an amine structure. The amine group in the amine structure may be a primary amine group, a secondary amine group, a tertiary amine group, or a quaternary amine group.

The structural unit having an amine structure may be a structural unit having an amide structure. The amide structure refers to a structure having -C(=O)-NH-.

The polyvinyl acetal resin preferably has an amine structure or an amide structure in a side chain. In this case, the amine structure or the amide structure may be directly bonded to the carbon atom constituting a main chain of the polyvinyl acetal resin, or may be bonded to the main chain via a linking group such as an alkylene group.

The content of the structural unit having an imine structure, the content of the structural unit having an imide structure, the content of the structural unit having an amine structure, and the content of the structural unit having an amide structure can be measured by $^1$H-NMR (nuclear magnetic resonance spectrum).

<Vinyl compound A>

Hereinafter, the vinyl compound A will be described in more detail.

The vinyl compound A is preferably a (meth)acrylic acid ester or a poly (meth) acrylic acid ester resin. In particular, it is preferable that the synthetic resin X is a copolymer obtained by graft-copolymerizing a polyvinyl acetal resin with a (meth)acrylic acid ester or a poly(meth)acrylic acid ester resin which is a polymer thereof.

The poly(meth)acrylic acid ester resin can be obtained by polymerizing the (meth)acrylic acid ester or by polymerizing the (meth)acrylic acid ester with the other monomers.

Examples of the (meth)acrylic acid ester include (meth)acrylic acid alkyl ester, (meth)acrylic acid cyclic alkyl ester, (meth)acrylic acid aryl ester, (meth)acryl amides, polyethylene glycol (meth)acrylates, phosphorylcholine (meth) acrylate, and the like.

Examples of the (meth)acrylic acid alkyl ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate, isotetradecyl (meth)acrylate, and the like.

The (meth)acrylic acid alkyl ester may be substituted with a substituent such as an alkoxy group having 1 to 3 carbon atoms and a tetrahydrofurfuryl group. Examples of such (meth)acrylic acid alkyl ester include methoxyethyl acrylate, tetrahydrofurfuryl acrylate, and the like.

Examples of the (meth)acrylic acid cyclic alkyl ester include cyclohexyl (meth)acrylate, isobornyl (meth)acrylate, and the like.

Examples of the (meth)acrylic acid aryl ester include phenyl (meth)acrylate, benzyl (meth)acrylate, and the like.

Examples of the (meth)acrylamides include (meth)acrylamide, N-isopropyl (meth)acrylamide, N-tert-butyl (meth)acrylamide, N,N'-dimethyl (meth)acrylamide, (3-(meth)acrylamide propyl)trimethylammonium chloride, 4-(meth)acryloylmorpholine, 3-(meth)acryloyl-2-oxazolidinone, N-[3-(dimethylamino)propyl] (meth)acrylamide, N-(2-hydroxyethyl) (meth)acrylamide, N-methylol(meth)acrylamide, 6-(meth)acrylamide hexane acid, and the like.

Examples of the polyethylene glycol (meth)acrylates include methoxy-polyethylene glycol (meth)acrylate, ethoxy-polyethylene glycol (meth)acrylate, hydroxy-polyethylene glycol (meth)acrylate, methoxy-diethylene glycol (meth)acrylate, ethoxy-diethylene glycol (meth)acrylate, hydroxy-diethylene glycol (meth)acrylate, methoxytriethylene glycol (meth)acrylate, ethoxy-triethylene glycol (meth)acrylate, hydroxy-triethylene glycol (meth)acrylate, and the like.

Examples of the phosphorylcholine (meth)acrylate include 2-(meth)acryloyloxyethyl phosphorylcholine and the like.

As the other monomer copolymerized with the (meth)acrylic acid ester, a vinyl compound is preferably used. Examples of the vinyl compound include ethylene, allylamine, vinylpyrrolidone, vinylimidazole, maleic anhydride, maleimide, itaconic acid, (meth)acrylic acid, vinylamine, (meth)acrylic acid ester, and the like. As the vinyl compound, only one type may be used, or two or more types may be used in combination.

In addition, in this specification, " (meth) acrylic" means "acrylic" or "methacrylic", and "(meth)acrylate" means "acrylate" or "methacrylate".

In the present invention, the synthetic resin X may be a copolymer of a resin having a poly(meth)acrylic acid ester skeleton and other vinyl compound as long as the phase-separated structure of the present invention can be formed.

As the other vinyl compound in this case, ethylene, allylamine, vinylpyrrolidone, maleic anhydride, maleimide, itaconic acid, (meth)acrylic acid, vinylamine, or other (meth)acrylic acid ester having a different SP value can be used.

(Synthetic resin X having peptide portion)

The cell culture scaffold material contains synthetic resin X having a peptide portion. The synthetic resin X having a peptide portion can be obtained by reacting the synthetic resin X with a linker and a peptide. The synthetic resin X having a peptide portion is preferably a peptide-conjugated polyvinyl acetal resin having a polyvinyl acetal resin portion, a linker portion, and a peptide portion, and more preferably a peptide-conjugated polyvinyl butyral resin having a polyvinyl butyral resin portion, a linker portion, and a peptide portion. As the synthetic resin X having a peptide portion, only one type may be used, or two or more types may be used in combination.

The peptide portion is preferably composed of 3 or more amino acids, more preferably composed of 4 or more amino acids and further preferably composed of 5 or more amino acids, and is preferably composed of 10 or less amino acids and more preferably composed of 6 or less amino acids. When the number of amino acids constituting the peptide portion is the above lower limit or more and the above upper limit or less, the adhesiveness to the cells after seeding can be further enhanced, and the proliferation rate of cells can be further enhanced.

The peptide portion preferably has a cell-adhesive amino acid sequence. The cell-adhesive amino acid sequence refers to an amino acid sequence whose cell adhesion activity has been confirmed by phage display method, sepharose beads method, or plate coating method. As the phage display method, for example, a method described in "The Journal of Cell Biology, Volume 130, Number 5, September 1995 1189-1196" can be used. As the sepharose beads method, for example, a method described in "Protein, Nucleic Acid and Enzyme, Vol.45 No.15 (2000) 2477" can be used. As the plate coating method, for example, a method described in "Protein, Nucleic Acid and Enzyme, Vol.45 No.15 (2000) 2477" can be used.

Examples of the cell-adhesive amino acid sequence include RGD sequence (Arg-Gly-Asp), YIGSR sequence (Tyr-Ile-Gly-Ser-Arg), PDSGR sequence (Pro-Asp-Ser-Gly-Arg), HAV sequence (His-Ala-Val), ADT sequence (Ala-Asp-Thr), QAV sequence (Gln-Ala-Val), LDV sequence (Leu-Asp-Val), IDS sequence (Ile-Asp-Ser), REDV sequence (Arg-Glu-Asp-Val), IDAPS sequence (Ile-Asp-Ala-Pro-Ser), KQAGDV sequence (Lys-Gln-Ala-Gly-Asp-Val), TDE sequence (Thr-Asp-Glu), and the like. In addition, examples of the cell-adhesive amino acid sequence include sequences described in "Medicina Philosophica, Vol. 9, No. 7, pp. 527-535, 1990" and "Journal of Osaka Women's and Children's Hospital, Vol. 8, No. 1, pp. 58-66, 1992", and the like. The peptide portion may have only one type of cell-adhesive amino acid sequence, or may have two or more types.

The cell-adhesive amino acid sequence preferably has at least one of the above-mentioned cell-adhesive amino acid sequences, more preferably has at least an RGD sequence, a YIGSR sequence or a PDSGR sequence, and further preferably has at least an RGD sequence represented by the following formula (1). In this case, the adhesiveness to the cells after seeding can be further enhanced, and the proliferation rate of cells can be further enhanced.

Arg-Gly-Asp-X ... Formula (1)

In Formula (1) above, X represents Gly, Ala, Val, Ser, Thr, Phe, Met, Pro, or Asn.

The peptide portion may be linear or may have a cyclic peptide skeleton. The cyclic peptide skeleton is a cyclic skeleton composed of a plurality of amino acids. From the viewpoint of more effectively exhibiting the effect of the present invention, the cyclic peptide skeleton is preferably composed of 4 or more amino acids, preferably composed of 5 or more amino acids, and preferably composed of 10 or less amino acids.

In the synthetic resin X having a peptide portion, the content of the peptide portion is preferably 0.1 mol% or more, more preferably 1 mol% or more, further preferably 5 mol% or more, and particularly preferably 10 mol% or more. In the synthetic resin X having a peptide portion, the content of the peptide portion is preferably 60 mol% or less, more preferably 50 mol% or less, further preferably 35 mol% or less, and particularly preferably 25 mol% or less. When the content of the peptide portion is the above lower limit or more, a phase-separated structure can be even more easily formed. When the content of the peptide portion is the above lower limit or more, the adhesiveness to the cells after seeding can be further enhanced, and the proliferation rate of cells can be further enhanced. Further, when the content of the peptide portion is the above upper limit or less, production cost can be suppressed. The content (mol%) of the peptide portion is the amount of substance of the peptide portion with respect to the total of the amount of substance of structural units constituting the synthetic resin X having a peptide portion.

The content of the peptide portion can be measured by FT-IR or LC-MS.

From the viewpoint of further enhancing the adhesiveness to the cells after seeding and further enhancing the proliferation rate of cells, in the synthetic resin X having a peptide portion, it is preferable that the second phase has a peptide portion, and it is more preferable that the peptide portion has the cell-adhesive amino acid sequence. It is preferable that the second phase is formed by the peptide portion of the synthetic resin X having a peptide portion. In this case, it is preferable that the second phase having a peptide portion is an island part. However, the first phase may have a peptide portion, and the second phase having a peptide portion may be a sea part.

In the synthetic resin X having a peptide portion, it is preferable that the synthetic resin X part and the peptide portion are bonded via a linker. That is, the synthetic resin X having a peptide portion is preferably a synthetic resin X having a peptide portion and a linker portion. As the linker, only one type may be used, or two or more types may be used in combination.

The linker is preferably a compound having a functional group capable of condensing with the carboxyl group or amino group of the peptide. Examples of the functional group capable of condensing with the carboxyl group or amino group of the peptide include a carboxyl group, a thiol group, an amino group, and the like. From the viewpoint of well reacting with a peptide, the linker is preferably a compound having a carboxyl group. As the linker, the above-mentioned vinyl compound A can also be used.

Examples of the linker having a carboxyl group include (meth)acrylic acid, a carboxyl group-containing acrylamide, and the like. By using a carboxylic acid having a polymerizable unsaturated group (carboxylic acid monomer) as the linker having a carboxyl group, the carboxylic acid monomer can be polymerized by graft polymerization when the linker and the synthetic resin X are reacted, so that the number of the carboxyl groups capable of reacting with a peptide can be increased.

[Cell culture scaffold material]

The cell culture scaffold material contains the synthetic resin X. From the viewpoint of effectively exhibiting the effect of the present invention and enhancing productivity, the content of the synthetic resin X in 100% by weight of the cell culture scaffold material is preferably 90% by weight or more, more preferably 95% by weight or more, further preferably 97.5% by weight or more, particularly preferably 99% by weight or more, and most preferably 100% by weight (whole amount). Therefore, it is most preferable that the cell culture scaffold material is the synthetic resin X. When the content of the synthetic resin X is the above lower limit or more, the effect of the present invention can be even more effectively exhibited.

The cell culture scaffold material may contain components other than the synthetic resin X. Components other than the synthetic resin X include polyolefin resins, polyether resins, polyvinyl alcohol resins, polyesters, epoxy resins, polyamide resins, polyimide resins, polyurethane resins, polycarbonate resins, polysaccharides, celluloses, polypeptides, synthetic peptides, and the like.

From the viewpoint of effectively exhibiting the effect of the present invention, the smaller the content of the components other than the synthetic resin X, the better. The content of the component in 100% by weight of the cell culture scaffold material is preferably 10% by weight or less, more preferably 5% by weight or less, further preferably 2.5% by weight or less, particularly preferably 1% by weight or less, and most preferably 0% by weight (not contained). Therefore, it is most preferable that the cell culture scaffold material does not contain any component other than the synthetic resin X.

It is preferable that the cell culture scaffold material does not substantially contain animal-derived raw materials. Since it does not contain animal-derived raw materials, it is possible to provide a cell culture scaffold material that is highly safe and has little variation in quality during production. In addition, the phrase "does not substantially contain animal-derived raw materials" means that the animal-derived raw materials in the cell culture scaffold material are 3% by weight or less. In the cell culture scaffold material, the animal-derived raw materials in the cell culture scaffold material are preferably 1% by weight or less, and more preferably 0% by weight. That is, it is more preferable that the cell culture scaffold material does not contain any animal-derived raw materials.

(Cell culture using cell culture scaffold material)

The cell culture scaffold material is used for culturing cells. The cell culture scaffold material is used as a scaffold for cells when culturing the cells. Therefore, a resin film formed of the cell culture scaffold material of the present invention is used for culturing cells, and is also used as a scaffold for cells when culturing the cells.

Examples of the cells include cells of animals such as human, mouse, rat, pig, cow and monkey. In addition, examples of the cells include somatic cells and the like, and examples thereof include stem cells, progenitor cells, mature cells, and the like. The somatic cells may be cancer cells.

Examples of the mature cells include nerve cells, cardiomyocytes, retinal cells, hepatocytes, and the like.

Examples of the stem cells include mesenchymal stem cells (MSCs), iPS cells, ES cells, Muse cells, embryonic cancer cells, embryonic germ cells, mGS cells, and the like.

(Form of cell culture scaffold material)

The resin film of the present invention is formed of a cell culture scaffold material. The resin film is formed by using a cell culture scaffold material. The resin film is preferably a film-like cell culture scaffold material. The resin film is preferably a film-like material made of cell culture scaffold material.

In this specification, particles, fibers, a porous body, or a film containing the cell culture scaffold material are also provided. In this case, the form of the cell culture scaffold material is not particularly limited, and may be particles, fibers, a porous body, or a film. The particles, fibers, porous body, or film may contain components other than the cell culture scaffold material.

The film containing the cell culture scaffold material is preferably used for plane culture (two-dimensional culture) of cells. In addition, particles, fibers, or a porous body containing the cell culture scaffold material are preferably used for three-dimensional culture of cells.

(Cell culture carrier)

The present invention also relates to a cell culture carrier in which the resin film is arranged on a surface of the carrier. The cell culture carrier of the present invention can be obtained, for example, by arranging the resin film on the surface of the carrier by coating or the like. The form of the carrier may be particles, fibers, a porous body, or a film. That is, the cell culture carrier of the present invention may be in the form of particles, fibers, a porous body, or a film. The cell culture carrier of the present invention may contain components other than the carrier and the resin film.

(Cell culture vessel)

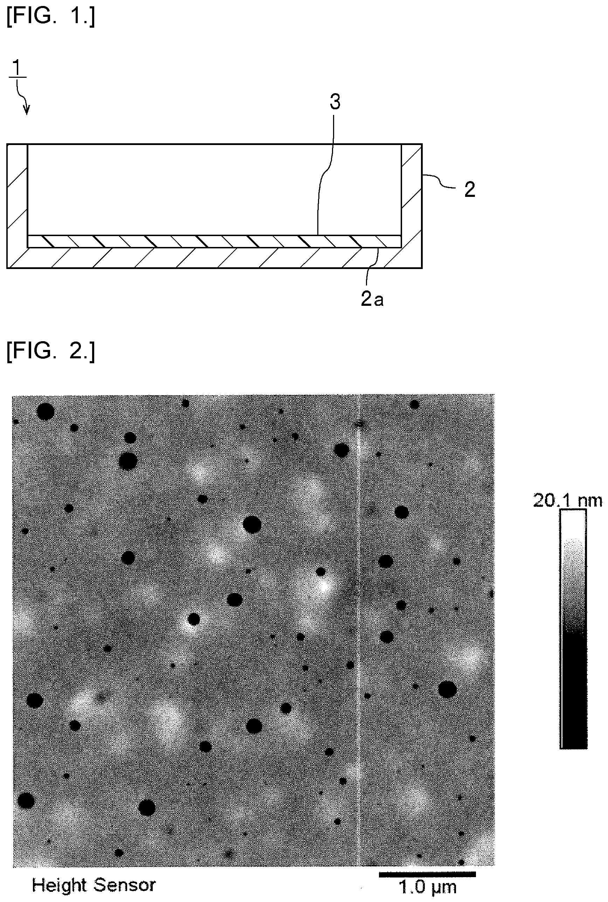

The present invention also relates to a cell culture vessel including the resin film in at least a part of the cell culture area. Fig. 1 is a cross-sectional view schematically showing a cell culture vessel according to an embodiment of the present invention.

A cell culture vessel 1 includes a vessel body 2 and a resin film 3 formed of a cell culture scaffold material. The resin film 3 is arranged on a surface 2a of the vessel body 2. The resin film 3 is arranged on a bottom surface of the vessel body 2. Cells can be cultured in plane by adding a liquid medium to the cell culture vessel 1 and seeding cells on a surface of the resin film 3.

The vessel body may include a first vessel body, and a second vessel body such as a cover glass on the bottom surface of the first vessel body. The first vessel body and the second vessel body may be separable. In this case, a resin film formed of the cell culture scaffold material may be arranged on the surface of the second vessel body.

As the vessel body, a conventionally known vessel body (vessel) can be used. Shape and size of the vessel body are not particularly limited.

Examples of the vessel body include a cell culture plate provided with one or a plurality of wells (holes), a cell culture flask, and the like. The number of wells in the plate is not particularly limited. The number of wells is not particularly limited, and examples thereof include 2, 4, 6, 12, 24, 48, 96, 384, and the like. Shape of the well is not particularly limited, and examples thereof include a perfect circle, an ellipse, a triangle, a square, a rectangle, a pentagon, and the like. Shape of the bottom surface of the well is not particularly limited, and examples thereof include a flat bottom, a round bottom, unevenness, and the like.

Material of the vessel body is not particularly limited, and examples thereof include resins, metals, and inorganic materials. Examples of the resin include polystyrene, polyethylene, polypropylene, polycarbonate, polyester, polyisoprene, cycloolefin polymer, polyimide, polyamide, polyamideimide, (meth)acrylic resin, epoxy resin, silicone, and the like. Examples of the metal include stainless steel, copper, iron, nickel, aluminum, titanium, gold, silver, platinum, and the like. Examples of the inorganic material include silicon oxide (glass), aluminum oxide, titanium oxide, zirconium oxide, iron oxide, silicon nitride, and the like.

**EXAMPLES**

Next, the present invention will be clarified by way of specific examples and comparative examples of the present invention. The present invention is not limited to the following examples.

The following synthetic resins were synthesized as raw materials for cell culture scaffold material.

(Example 1)

A reactor equipped with a stirrer was charged with 2700 mL of ion-exchanged water, 300 parts by weight of polyvinyl alcohol with an average degree of polymerization of 1700 and a degree of saponification of 98 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 148 parts by weight of n-butyraldehyde was added to precipitate a white particulate polyvinyl butyral resin. Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, then washed with water, and dried to obtain a polyvinyl butyral resin (PVB, SP value: 9.9) as a polyvinyl acetal resin. Ninety parts by weight of the obtained polyvinyl butyral resin was dissolved in tetrahydrofuran so as to be a 1% by weight solution, and 5 parts by weight of Irgacre184 as an initiator, 2 parts by weight of N-vinylpyrrolidone (SP value: 11.7) and 8 parts by weight of n-lauryl methacrylate (SP value: 8.2) were added thereto to carry out graft polymerization to obtain a synthetic resin. The obtained synthetic resin has a degree of acetalization (degree of butyralization) of 69 mol%, an amount of hydroxyl groups of 27.5 mol%, a degree of acetylation of 2.0 mol%, a content of vinylpyrrolidone group of 0.3 mol%, and a content of n-lauryl methacrylate of 1.2 mol%.

(Examples 2 to 11 and Comparative Example 1)

Synthetic resins were obtained in the same manner as in Example 1 except that the weight ratios of the polyvinyl butyral resin, N-vinylpyrrolidone, and n-lauryl methacrylate were changed. Table 1, Table 2 and Table 4 show degrees of acetalization (degrees of butyralization), amounts of hydroxyl groups, degrees of acetylation, contents of vinylpyrrolidone groups, and contents of n-lauryl methacrylate of the synthetic resins obtained in Examples 2 to 11 and Comparative Example 1.

(Example 12)

A reactor equipped with a stirrer was charged with 2700 mL of ion-exchanged water, 300 parts by weight of polyvinyl alcohol with an average degree of polymerization of 1700 and a degree of saponification of 99 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 148 parts by weight of n-butyraldehyde was added thereto to precipitate a white particulate polyvinyl acetal resin (polyvinyl butyral resin). Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, and then the polyvinyl butyral resin was washed with water and dried to obtain a polyvinyl acetal resin (polyvinyl butyral resin, an average degree of polymerization of 1700, a degree of acetalization (degree of butyralization) of 70 mol%, an amount of hydroxyl groups of 27 mol%, and a degree of acetylation of 3 mol%).

Introduction of linker:

Nighty nine parts by weight of the obtained polyvinyl acetal resin and 1 part by weight of acrylic acid (linker) were dissolved in 300 parts by weight of THF and reacted in the presence of a photoradical polymerization initiator for 20 minutes under ultraviolet irradiation to graft-copolymerize a polyvinyl acetal resin with acrylic acid, thereby introducing the linker. One part by weight of the polyvinyl acetal resin into which the linker was introduced was dissolved in 19 parts by weight of butanol. The obtained solution (150 μL) was discharged onto a surface of a φ22 mm cover glass ("22 round No. 1" manufactured by Matsunami Glass Ind., Ltd.) subjected to dust removal with an air duster, rotated at 2000 rpm for 20 seconds using a spin coater, and then heated at 60°C for 60 minutes to obtain a resin film with a smooth surface.

Formation of peptide portion:

A linear peptide having an amino acid sequence of Gly-Arg-Gly-Asp-Ser (five amino acid residues, described as GRGDS in the table) was prepared. Ten parts by weight of this peptide and 1 part by weight of 1-ethyl-3-(3-dimethylaminopropyl)carbodiimide hydrochloride (condensing agent) were added to phosphate buffered saline containing neither calcium nor magnesium so that the final concentration of the peptide is 1 mM to prepare a peptide-containing solution. One part by weight of this peptide-containing liquid was added to a spin-coated resin film (polyvinyl acetal resin with a linker formed) and reacted to dehydrate and condense a carboxyl group of the linker and an amino group of Gly of the peptide. In this way, a peptide-conjugated polyvinyl acetal resin having a polyvinyl acetal resin portion, a linker portion and a peptide portion was prepared.

The obtained peptide-conjugated polyvinyl acetal resin had a degree of acetalization (degree of butyralization) of 69 mol%, an amount of hydroxyl groups of 27 mol%, a degree of acetylation of 3 mol%, a content of carboxyl groups of 0.1 mol%, and a content of peptide portion of 1.0 mol%.

(Example 13)

A peptide-conjugated polyvinyl acetal resin was prepared in the same manner as in Example 12 except that 85 parts by weight of the polyvinyl acetal resin and 15 parts by weight of acrylic acid (linker) were used in the introduction of linker, and the amount of the peptide added was changed to 15 parts by weight in the formation of peptide.

(Example 14)

A peptide-conjugated polyvinyl acetal resin was prepared in the same manner as in Example 12 except that 70 parts by weight of the polyvinyl acetal resin and 30 parts by weight of acrylic acid (linker) were used in the introduction of linker, and the amount of the peptide added was changed to 30 parts by weight in the formation of peptide.

(Example 15)

A peptide-conjugated polyvinyl acetal resin was prepared in the same manner as in Example 12 except that 67 parts by weight of the polyvinyl acetal resin and 33 parts by weight of acrylic acid (linker) were used in the introduction of linker, and the amount of the peptide added was changed to 33 parts by weight in the formation of peptide.

(Example 16)

A peptide-conjugated polyvinyl acetal resin was prepared in the same manner as in Example 12 except that 63 parts by weight of the polyvinyl acetal resin and 37 parts by weight of acrylic acid (linker) were used in the introduction of linker, and the amount of the peptide added was changed to 37 parts by weight in the formation of peptide.

(Example 17)

A peptide-conjugated polyvinyl acetal resin was prepared in the same manner as in Example 12 except that 30 parts by weight of polyvinyl acetal resin and 70 parts by weight of acrylic acid (linker) were used in the introduction of linker, and the amount of the peptide added was changed to 70 parts by weight in the formation of peptide.

(Comparative Example 2)

As the synthetic resin, a polystyrene resin was used as it was.

(Comparative Example 3)

A reactor equipped with a stirrer was charged with 2700 mL of ion-exchanged water, 300 parts by weight of polyvinyl alcohol with an average degree of polymerization of 1700 and a degree of saponification of 98 mol%, followed by dissolution by heating with stirring to obtain a solution. To the obtained solution, 35% by weight hydrochloric acid as a catalyst was added such that the concentration of hydrochloric acid became 0.2% by weight. Subsequently, temperature was adjusted to 15°C, and 22 parts by weight of n-butyraldehyde was added thereto with stirring. Then, 148 parts by weight of n-butyraldehyde was added to precipitate a white particulate polyvinyl butyral resin. Fifteen minutes after the precipitation, 35% by weight hydrochloric acid was added such that the concentration of hydrochloric acid became 1.8% by weight, and then the mixture was heated to 50°C and kept at 50°C for 2 hours. Next, the solution was cooled and neutralized, then washed with water, and dried to obtain a polyvinyl butyral resin (SP value: 9.9). That is, a polyvinyl butyral resin (synthetic resin) in which the vinyl compound was not copolymerized was obtained.

(Comparative Example 4)

Seventeen parts by weight of N-vinylpyrrolidone and 83 parts by weight of n-lauryl methacrylate were mixed to obtain a (meth)acrylic monomer solution. One part by weight of Irgacure184 (manufactured by BASF SE) was dissolved in the obtained (meth)acrylic monomer solution, and was applied onto a PET film. A poly(meth)acrylic acid ester resin solution was obtained by irradiating the coated material with light with a wavelength of 365 nm at an integrated light quantity of 2000 mJ/cm$^2$ using a UV conveyor device "ECS301G1" manufactured by Eye Graphics Co., Ltd.) at 25°C. The obtained poly(meth) acrylic acid ester resin solution was vacuum-dried at 80°C for 3 hours to obtain a synthetic resin as a poly(meth) acrylic acid ester resin.

(Reference Example A)

Preparation of scaffolding derived from natural product:
A Vitronectin (manufactured by Corning Incorporated) solution (1 ml) adjusted to 5 ug/ml in phosphate buffer (PBS) was added to a φ35 mm dish. A φ22 mm cover glass ("22 round No. 1" manufactured by Matsunami Glass Ind., Ltd.) was immersed therein and cured at 37°C for 1 hour, whereby scaffolding derived from a natural product in which Vitronectin (described as VTN in the table) was smoothly adsorbed on a surface was obtained.

Preparation of cell culture vessel:

A laminate of Vitronectin and the cover glass was arranged on a φ22 mm polystyrene dish to obtain a cell culture vessel. Since Vitronectin is denatured when dried and its adhesive performance is significantly reduced, the cell culture vessel was immersed in a PBS solution immediately after being prepared.

[Evaluation]

(Degree of acetalization and cationic group modification degree)

Degrees of acetalization and cationic group modification degrees of the synthetic resins obtained in Examples and Comparative Examples were determined by $^1$H-NMR (nuclear magnetic resonance spectrum) after dissolving the synthetic resin in DMSO-d6 (dimethylsulfoxide).

(Storage elastic modulus)

Storage elastic moduli at 25°C and 100°C of each scaffold material were measured by a dynamic viscoelasticity measuring device (manufactured by IT Keisoku Seigyo Co., Ltd., DVA-200) under tensile conditions at a frequency of 10 Hz and a temperature range of -150°C to 150°C at a heating rate of 5°C/min. The storage elastic moduli at 25°C and 100°C were obtained from a graph of the obtained tensile storage elastic modulus, and 25°C storage elastic modulus/100°C storage elastic modulus was calculated. The measurement was performed using a measurement sample with a length of 50 mm, a width of 5 to 20 mm, and a thickness of 0.1 to 1.0 mm, under conditions of 10 Hz, a strain of 0.1%, a temperature of -150°C to 150°C, and a heating rate of 5°C/min.

(Water swelling ratio)

A resin film (measurement sample) made of each scaffolding material with a length of 50 mm, a width of 10 mm, and a thickness of 0.05 mm to 0.15 mm was immersed in water at 25°C for 24 hours. Weights of the sample before and after immersion were measured, and Water swelling ratio = (Sample weight after immersion - Sample weight before immersion) / (Sample weight before immersion) $\times$ 100 (%) was calculated.

(Preparation of cell culture vessel)

In Examples 1 to 11 and Comparative Examples 1 to 4, a resin solution was obtained by dissolving 1 g of the obtained synthetic resin in 19 g of butanol. The obtained resin solution (150 μL) was discharged onto a φ22 mm cover glass (manufactured by Matsunami Glass Ind., Ltd., using 22 round No. 1 after removing dust with an air duster), and rotated at 2000 rpm for 20 seconds using a spin coater to obtain a smooth resin film. The obtained resin film together with the cover 26 glass was arranged on a φ22 mm polystyrene dish to obtain a cell culture vessel. In Examples 12 to 17, a laminate of the obtained peptide-conjugated polyvinyl acetal resin and the cover glass was arranged on a φ22 mm polystyrene dish to obtain a cell culture vessel.

(Surface free energy)

The surface free energy of the resin film obtained in the section of preparation of cell culture vessel was measured using a contact angle meter (DMo-701 manufactured by Kyowa Interface Science Co., Ltd.). A contact angle of pure water was obtained by dropping 1 μL of pure water onto the resin film and photographing a droplet image 30 seconds later. Further, a contact angle of diiodomethane was obtained by dropping 1 μL of diiodomethane onto the resin film, and photographing a droplet image 30 seconds later. From the obtained contact angles, dispersion term component $\gamma^d$ (dSFE) of surface free energy and dipole component $\gamma^p$ (pSFE) as a polar term component of surface free energy were calculated using the Kaelble-Uy theoretical formula.

(Phase separation parameter)

The resin film obtained in the section of preparation of cell culture vessel was observed with an atomic force microscope (AFM, manufactured by Bruker, product number "Dimension XR"). As a cantilever, SCAN ASYST AIR was used. As a result, as shown in Fig. 2, in the resin film of Example 3, a sea-island structure was observed in which the polyvinyl butyral resin portion as the first phase formed a sea part, and a resin portion having the (meth)acrylic acid ester and the vinyl compound (copolymer portion of N-vinylpyrrolidone and n-laurylmethacrylate) as the second phase formed island parts. Similarly, also in Examples 1 to 2, 4 to 11, a sea-island structure was observed in which the polyvinyl butyral resin portion as the first phase formed a sea part, and a resin portion having the (meth)acrylic acid ester and the vinyl compound (copolymer portion of N-vinylpyrrolidone and n-laurylmethacrylate) as the second phase formed island parts. Also in Examples 12 to 17, a sea-island structure was observed in which the polyvinyl butyral resin portion as the first phase formed a sea part and the peptide portion as the second phase formed island parts. On the other hand, in Comparative Examples 1 to 4, no phase-separated structure was observed.

In addition, the ratio of the surface area of the second phase to the entire surface (surface area fraction of the phase-separated structure), a ratio of the peripheral length to the area of the second phase (peripheral length/area), the number of the second phases as island parts (number of islands) and the average diameter of the island parts (average island size) were obtained by the above-mentioned method, using image analysis software (ImageJ) from the image obtained by the atomic force microscope.

(Cell proliferation rate)

Phosphate buffered saline (1 mL) was added to the obtained cell culture vessel, and the mixture was allowed to stand in an incubator at 37°C for 1 hour, then the phosphate buffered saline in the culture vessel was removed. Colonies of h-iPS cells 253G1 in a confluent state were added to a 35 mm dish, followed by addition of 1 mL of a 0.5 mM ethylenediamine/phosphate buffer solution, and the mixture was allowed to stand at room temperature for 2 minutes. Then, the ethylenediamine/phosphate buffer solution was removed, and a cell mass ($0.5 \times 10^5$ cells) crushed to 50 to 200 $\mu$m by pipetting with 1 mL of TeSRE8 medium was seeded in a culture vessel. In the presence of 1.7 mL of medium TeSR E8 (manufactured by STEMCELL Technologies Inc.) and 10 $\mu$M of ROCK-Inhibitor (Y27632), the cells were cultured in an incubator at 37°C and a $CO_2$ concentration of 5%. The medium (1 mL) was removed every 24 hours, and 1 mL of fresh TeSR E8 was added to replace the medium. A colonized cell mass after 5 days was exfoliated with 1.0 mL of TryPLE Express exfoliating solution, and the number of cells was counted using a cell counter (Nucleocounter NC-3000, manufactured by Chemometec).

A cell proliferation rate relative to Reference Example A was determined using the following formula.

Cell proliferation rate relative to Reference Example A (%) = (Number of cells in Examples or Comparative Examples)/ (Number of cells in Reference Example A) $\times$ 100

The cell proliferation rate was evaluated according to the following criteria.

[Evaluation criteria]

AAA ... Cell proliferation rate relative to Reference Example A is 70% or more
AA ... Cell proliferation rate relative to Reference Example A is 60% or more and less than 70%
A ... Cell proliferation rate relative to Reference Example A is 50% or more and less than 60%
B ... Cell proliferation rate relative to Reference Example A is 40% or more and less than 50%
C ... Cell proliferation rate relative to Reference Example A is 30% or more and less than 40%
D ... Cell proliferation rate relative to Reference Example A is less than 30%

The results are shown in Tables 1 to 4 below.

[Table 1]

| | | | Example 1 | Example 2 | Example 3 | Example 4 | Example 5 | Example 6 |
|---|---|---|---|---|---|---|---|---|
| Synthetic resin | Polyvinyl acetal resin | Degree of acetalization (mol%) | 69.0 | 63.0 | 49.0 | 39.9 | 35.0 | 28.0 |
| | | Amount of hydroxyl groups (mol%) | 27.5 | 25.2 | 19.6 | 16.0 | 14.0 | 11.2 |
| | | Amount of Acetyl groups (mol%) | 2.1 | 1.8 | 1.4 | 1.1 | 1.0 | 0.8 |
| | Vinyl compound A (Acrylic/Vinyl) | N-Vinylpyrrolidone (mol%) | 0.3 | 2.0 | 5.0 | 7.0 | 8.3 | 10.0 |
| | | n-Lauryl methacrylate (mol%) | 1.2 | 8.0 | 25.0 | 36.0 | 41.7 | 50.0 |
| Physical properties | Storage elastic modulus | 25°C Storage elastic modulus (Pa) | $2.2 \times 10^9$ | $1.8 \times 10^9$ | $1.9 \times 10^9$ | $1.3 \times 10^9$ | $9.6 \times 10^8$ | $9.0 \times 10^8$ |
| | | 100°C Storage elastic modulus (Pa) | $2.7 \times 10^6$ | $2.7 \times 10^6$ | $3.0 \times 10^6$ | $4.7 \times 10^6$ | $4.6 \times 10^6$ | $4.2 \times 10^6$ |
| | | 25°C Storage elastic modulus/ 100°C storage elastic modulus | $8.2 \times 10^2$ | $6.6 \times 10^2$ | $6.3 \times 10^2$ | $2.7 \times 10^2$ | $2.1 \times 10^2$ | $2.1 \times 10^2$ |
| | Water swelling ratio (%) | | 15 | 16 | 18 | 15 | 10 | 11 |
| | Surface free energy | Dispersion term component (dSFE) (mJ/m$^2$) | 35.8 | 36 | 36.8 | 43.5 | 44.2 | 44.5 |
| | | Polar term component (pSFE) (mJ/m$^2$) | 2.3 | 2 | 2.7 | 2.5 | 1.6 | 2 |
| Phase separation parameters | Surface area fraction of phase-separated structure (-) | | 0.015 | 0.1 | 0.3 | 0.43 | 0.5 | 0.6 |
| | Peripheral length/area (1/nm) | | 0.0800 | 0.0133 | 0.0160 | 0.0286 | 0.0333 | 0.0267 |
| | Number of islands (pieces/$\mu$m$^2$) | | 2 | 2 | 4 | 10 | 70 | 60 |
| | Average island size (nm) | | 50 | 300 | 250 | 140 | 120 | 150 |
| Culture evaluation | Cell proliferation rate relative to Reference Example A (%) | | 30 | 33 | 51 | 69 | 65 | 55 |
| | Cell culture performance | | C | C | A | AA | AA | A |

[Table 2]

| | | | Example 7 | Example 8 | Example 9 | Example 10 | Example 11 |
|---|---|---|---|---|---|---|---|
| Synthetic resin | Polyvinyl acetal resin | Degree of acetalization (mol%) | 23.1 | 18.5 | 14.0 | 7.0 | 3.5 |
| | | Amount of hydroxyl groups (mol%) | 10.0 | 6.0 | 5.5 | 2.7 | 0.8 |
| | | Amount of Acetyl groups (mol%) | 0.7 | 0.5 | 0.5 | 0.3 | 0.2 |
| | Vinyl compound A (Acrylic/Vinyl) | N-Vinylpyrrolidone (mol%) | 11.0 | 12.5 | 13.0 | 15.0 | 16.0 |
| | | n-Lauryl methacrylate (mol%) | 55.0 | 62.5 | 67.0 | 75.0 | 79.0 |
| Physical properties | Storage elastic modulus | 25°C Storage elastic modulus (Pa) | $9.2 \times 10^8$ | $8.0 \times 10^8$ | $6.0 \times 10^8$ | $4.8 \times 10^8$ | $3.0 \times 10^6$ |
| | | 100°C Storage elastic modulus (Pa) | $3.8 \times 10^6$ | $3.8 \times 10^6$ | $2.6 \times 10^6$ | $2.4 \times 10^6$ | $2.6 \times 10^6$ |
| | | 25°C Storage elastic modulus/ 100°C storage elastic modulus | $2.4 \times 10^2$ | $2.1 \times 10^2$ | $2.3 \times 10^2$ | $2.0 \times 10^2$ | $1.2 \times 10^2$ |
| | Water swelling ratio (%) | | 12 | 10 | 9 | 10 | 22 |
| | Surface free energy | Dispersion term component (dSFE) (mJ/m$^2$) | 43.6 | 44.0 | 44.7 | 45.0 | 45.5 |
| | | Polar term component (pSFE) (mJ/m$^2$) | 2.2 | 2.0 | 2.3 | 1.7 | 1.2 |
| Phase separation parameters | Surface area fraction of phase-separated structure (-) | | 0.66 | 0.75 | 0.8 | 0.9 | 0.95 |
| | Peripheral length/area (1/nm) | | 0.0160 | 0.0027 | 0.0016 | 0.0013 | 0.0013 |
| | Number of islands (pieces/$\mu$m$^2$) | | 20 | 2 | 4 | 2 | 1 |
| | Average island size (nm) | | 250 | 1500 | 2500 | 3000 | 3200 |
| Culture evaluation | Cell proliferation rate relative to Reference Example A (%) | | 61 | 41 | 48 | 35 | 34 |
| | Cell culture performance | | AA | B | B | C | C |

[Table 3]

| | | | Example 12 | Example 13 | Example 14 | Example 15 | Example 16 | Example 17 |
|---|---|---|---|---|---|---|---|---|
| Synthetic resin | Polyvinyl acetal resin | Degree of acetalization (mol%) | 69.3 | 64.3 | 64.0 | 57.5 | 54.0 | 24.0 |
| | | Amount of hydroxyl groups (mol%) | 26.7 | 25.0 | 22.0 | 23.0 | 21.0 | 9.0 |
| | | Amount of Acetyl groups (mol%) | 2.9 | 3.0 | 3.0 | 3.0 | 3.0 | 1.0 |
| | Vinyl compound A (Acrylic/Vinyl) | N-Vinylpyrrolidone (mol%) | - | - | - | - | - | - |
| | | n-Lauryl methacrylate (mol%) | - | - | - | - | - | - |
| | | Acrylic acid (mol%) | 0.1 | 0.7 | 1.0 | 1.5 | 2.0 | 6.0 |
| Peptide portion | GRGDS | Content (mol%) | 1.0 | 7.0 | 10.0 | 15.0 | 20.0 | 60.0 |
| Physical properties | Storage elastic modulus | 25°C Storage elastic modulus (Pa) | $2.5 \times 10^9$ | $2.0 \times 10^9$ | $1.9 \times 10^9$ | $2.2 \times 10^9$ | $2.0 \times 10^9$ | $2.5 \times 10^9$ |
| | | 100°C Storage elastic modulus (Pa) | $3.0 \times 10^6$ | $4.0 \times 10^6$ | $3.5 \times 10^6$ | $3.4 \times 10^6$ | $1.0 \times 10^6$ | $2.0 \times 10^6$ |
| | | 25°C Storage elastic modulus/ 100°C storage elastic modulus | $8.3 \times 10^2$ | $5.0 \times 10^2$ | $5.1 \times 10^2$ | $6.5 \times 10^2$ | $2.0 \times 10^3$ | $1.3 \times 10^2$ |
| | Water swelling ratio (%) | | 4 | 6 | 7 | 10 | 15 | 18 |
| | Surface free energy | Dispersion term component (dSFE) (mJ/m$^2$) | - | - | - | - | - | - |
| | | Polar term component (pSFE) (mJ/m$^2$) | - | - | - | - | - | - |
| Phase separation parameters | | Surface area fraction of phase-separated structure (-) | 0.079 | 0.100 | 0.353 | 0.495 | 0.589 | 0.785 |
| | | Peripheral length/area (1/nm) | 0.2000 | 0.1000 | 0.0400 | 0.0133 | 0.0080 | 0.0040 |
| | | Number of islands (pieces/µm$^2$) | 250 | 80 | 45 | 7 | 3 | 1 |
| | | Average island size (nm) | 20 | 40 | 100 | 300 | 500 | 1000 |
| Culture evaluation | | Cell proliferation rate relative to Reference Example A (%) | 69 | 95 | 98 | 64 | 59 | 59 |
| | | Cell culture performance | AA | AAA | AAA | AA | A | A |

[Table 4]

| | | | Comparative Example 1 | Comparative Example 2 | Comparative Example 3 | Comparative Example 4 | Reference Example A |
|---|---|---|---|---|---|---|---|
| Synthetic resin | Polyvinyl acetal resin | Degree of acetalization (mol%) | 1.4 | - | 70.0 | - | VTN |
| | | Amount of hydroxyl groups (mol%) | 0.56 | - | 28.0 | - | |
| | | Amount of Acetyl groups (mol%) | 0.04 | - | 2.0 | - | |
| | Vinyl compound A (Acrylic/Vinyl) | N-Vinylpyrrolidone (mol%) | 16.0 | - | - | 17.0 | |
| | | n-Lauryl methacrylate (mol%) | 82.0 | - | - | 83.0 | |
| Physical properties | Storage elastic modulus | 25°C Storage elastic modulus (Pa) | $1.6 \times 10^8$ | $2.3 \times 10^9$ | $2.2 \times 10^9$ | $1.2 \times 10^6$ | - |
| | | 100°C Storage elastic modulus (Pa) | $6.0 \times 10^5$ | $2.1 \times 10^8$ | $2.7 \times 10^6$ | $9.0\text{-}10^3$ | - |
| | | 25°C Storage elastic modulus/ 100°C storage elastic modulus | $2.6 \times 10^2$ | $1.1 \times 10^1$ | $8.2 \times 10^2$ | $1.3 \times 10^2$ | - |
| | Water swelling ratio (%) | | 26 | 0 | 16 | 55 | - |
| | Surface free energy | Dispersion term component (dSFE) (mJ/m$^2$) | 48 | 29.4 | 32.8 | 50 | - |
| | | Polar term component (pSFE) (mJ/m$^2$) | 1 | 20.6 | 3.6 | 0.5 | - |
| Phase separation parameters | Surface area fraction of phase-separated structure (-) | | 0.98 | 0 | 0 | 0 | - |
| | Peripheral length/area (1/nm) | | 0.0011 | - | - | - | - |
| | Number of islands (pieces/$\mu$m$^2$) | | 0 | 0 | 0 | 0 | - |
| | Average island size (nm) | | 3500 | 0 | 0 | 0 | - |
| Culture evaluation | Cell proliferation rate relative to Reference Example A (%) | | 18 | 0 | 20 | 0 | - |
| | Cell culture performance | | D | D | D | D | - |

**EXPLANATION OF SYMBOLS**

1: Cell culture vessel
2: Vessel body
2a: Surface
3: Resin film

**Claims**

1. A resin film formed of a cell culture scaffold material,

 the cell culture scaffold material containing a synthetic resin,
 the synthetic resin being a peptide-conjugated polyvinyl acetal resin having a polyvinyl acetal resin portion, a linker portion, and a peptide portion,
 the resin film having a phase-separated structure including at least a first phase and a second phase, and
 a ratio of the surface area of one of the first phase and the second phase to the entire surface being 0.01 or more and 0.95 or less.

2. The resin film according to claim 1, wherein the ratio of the peripheral length to the area of the second phase (peripheral length/area) is 0.001 (1/nm) or more and 0.40 (1/nm) or less.

3. The resin film according to claim 1 or 2, wherein

 the phase-separated structure is a sea-island structure,
 the first phase is a sea part, and
 the second phase is an island part.

4. The resin film according to claim 3, wherein the number of the second phase as an island part is 1 piece/$\mu$m$^2$ or more and 5,000 pieces/$\mu$m$^2$ or less.

5. The resin film according to any one of claims 1 to 4, wherein the phase-separated structure is composed of a phase-separated structure within a molecule of the synthetic resin.

6. The resin film according to any one of claims 1 to 5, wherein

 the synthetic resin has a cationic functional group, and
 the content of the cationic functional group contained in a structural unit of the synthetic resin is 0.2 mol% or more and 50 mol% or less.

7. The resin film according to any one of claims 1 to 5, wherein the second phase has the peptide portion.

8. The resin film according to any one of claims 1 to 7, wherein the peptide portion has a cell-adhesive amino acid sequence.

9. The resin film according to any one of claims 1 to 8, which has a water swelling ratio of 50% or less.

10. The resin film according to any one of claims 1 to 9, which has a storage elastic modulus at 100°C of $1.0 \times 10^4$ Pa or more and $1.0 \times 10^8$ Pa or less, and a ratio of a storage elastic modulus at 25°C to a storage elastic modulus at 100°C ((storage elastic modulus at 25°C)/(storage elastic modulus at 100°C)) of $1.0 \times 10^1$ or more and $1.0 \times 10^5$ or less.

11. The resin film according to any one of claims 1 to 10, wherein the cell culture scaffold material does not substantially contain animal-derived raw materials.

12. A cell culture carrier, comprising:

a carrier; and
the resin film according to any one of claims 1 to 11,
the resin film being arranged on a surface of the carrier.

**13.** A cell culture vessel, comprising:

a vessel body; and
the resin film according to any one of claims 1 to 11,
the resin film being arranged on a surface of the vessel body.

**Patentansprüche**

**1.** Harzfilm aus einem Zellkulturgerüstmaterial,

wobei das Zellkulturgerüstmaterial ein Kunstharz enthält,
wobei es sich bei dem Kunstharz um ein Peptid-konjugiertes Polyvinylacetalharz mit einem Polyvinylacetalharzanteil, einem Linkeranteil und einem Peptidanteil handelt,
wobei der Harzfilm eine phasengetrennte Struktur mit mindestens einer ersten Phase und einer zweiten Phase aufweist und
das Verhältnis der Oberfläche der ersten Phase und der zweiten Phase zur Gesamtoberfläche 0,01 oder mehr und 0,95 oder weniger beträgt.

**2.** Harzfilm nach Anspruch 1, wobei das Verhältnis der Umfangslänge zur Fläche der zweiten Phase (Umfangslänge/Fläche) 0,001 (1/nm) oder mehr und 0,40 (1/nm) oder weniger beträgt.

**3.** Harzfilm nach Anspruch 1 oder 2, wobei

die phasengetrennte Struktur eine Meer-Insel-Struktur ist,
die erste Phase ein Meerteil und
die zweite Phase ein Inselteil ist.

**4.** Harzfilm nach Anspruch 3, wobei die Anzahl der zweiten Phase als Inselteil 1 Stück/$\mu$m$^2$ oder mehr und 5.000 Stück/$\mu$m$^2$ oder weniger beträgt.

**5.** Harzfilm nach einem der Ansprüche 1 bis 4, wobei die phasengetrennte Struktur aus einer phasengetrennten Struktur innerhalb eines Moleküls des Kunstharzes besteht.

**6.** Harzfilm nach einem der Ansprüche 1 bis 5, wobei

das Kunstharz eine kationische funktionelle Gruppe aufweist und
der Gehalt der kationischen funktionellen Gruppe in einer Struktureinheit des Kunstharzes 0,2 Mol-% oder mehr und 50 Mol-% oder weniger beträgt.

**7.** Harzfilm nach einem der Ansprüche 1 bis 5, wobei die zweite Phase den Peptidanteil aufweist.

**8.** Harzfilm nach einem der Ansprüche 1 bis 7, wobei der Peptidanteil eine zelladhäsive Aminosäuresequenz aufweist.

**9.** Harzfilm nach einem der Ansprüche 1 bis 8 mit einem Wasserquellverhältnis von 50% oder weniger.

**10.** Harzfilm nach einem der Ansprüche 1 bis 9, der einen Speichermodul bei 100°C von $1,0 \times 10^4$ Pa oder mehr und $1,0 \times 10^8$ Pa oder weniger aufweist und
ein Verhältnis des Speichermoduls bei 25°C zum Speichermodul bei 100°C ((Speichermodul bei 25°C)/(Speichermodul bei 100°C)) von $1,0 \times 10^1$ oder mehr und $1,0 \times 10^5$ oder weniger aufweist.

**11.** Harzfilm nach einem der Ansprüche 1 bis 10, wobei das Zellkulturgerüstmaterial im Wesentlichen keine tierischen Rohstoffe enthält.

**12.** Zellkulturträger, umfassend:

einen Träger; und
den Harzfilm nach einem der Ansprüche 1 bis 11,
wobei der Harzfilm auf einer Oberfläche des Trägers angeordnet ist.

**13.** Zellkulturgefäß, umfassend:

einen Gefäßkörper; und
den Harzfilm nach einem der Ansprüche 1 bis 11,
wobei der Harzfilm auf einer Oberfläche des Gefäßkörpers angeordnet ist.

**Revendications**

**1.** Film de résine formé d'un matériau de support de culture cellulaire,

le matériau de support de culture cellulaire contenant une résine synthétique,
la résine synthétique étant une résine de polyacétal de vinyle conjuguée à un peptide ayant une portion de résine de polyacétal de vinyle, une portion de lieur et une portion de peptide,
le film de résine ayant une structure à phases séparées incluant au moins une première phase et une seconde phase, et
un rapport de l'aire de surface d'une phase parmi la première phase et la seconde phase sur la surface totale étant de 0,01 ou plus et de 0,95 ou moins.

**2.** Film de résine selon la revendication 1, dans lequel le rapport de la longueur périphérique sur l'aire de la seconde phase (longueur périphérique/aire) est de 0,001 (1/nm) ou plus et de 0,40 (1/nm) ou moins.

**3.** Film de résine selon la revendication 1 ou 2, dans lequel

la structure à phases séparées est une structure mer-îlot,
la première phase est une partie de mer, et
la seconde phase est une partie d'îlot.

**4.** Film de résine selon la revendication 3, dans lequel le nombre de la seconde phase faisant office de partie d'îlot est de 1 pièce/$\mu m^2$ ou plus et de 5 000 pièces/$\mu m^2$ ou moins.

**5.** Film de résine selon l'une quelconque des revendications 1 à 4, dans lequel la structure à phases séparées est composée d'une structure à phases séparées à l'intérieur d'une molécule de la résine synthétique.

**6.** Film de résine selon l'une quelconque des revendications 1 à 5, dans lequel

la résine synthétique possède un groupe fonctionnel cationique, et
la teneur en groupe fonctionnel cationique contenu dans un motif structural de la résine synthétique est de 0,2 % en mole ou plus et de 50 % en mole ou moins.

**7.** Film de résine selon l'une quelconque des revendications 1 à 5, dans lequel la seconde phase a la portion de peptide.

**8.** Film de résine selon l'une quelconque des revendications 1 à 7, dans lequel la portion de peptide a une séquence d'acides aminés d'adhésion cellulaire.

**9.** Film de résine selon l'une quelconque des revendications 1 à 8, qui a un taux de gonflement dans l'eau de 50 % ou moins.

**10.** Film de résine selon l'une quelconque des revendications 1 à 9, qui a un module de cisaillement élastique à 100 °C de $1,0 \times 10^4$ Pa ou plus et de $1,0 \times 10^8$ Pa ou moins, et un rapport d'un module de cisaillement élastique à 25°C sur un module de cisaillement élastique à 100 °C ((module de cisaillement élastique à 25 °C)/(module de cisaillement élastique à 100 °C)) de $1,0 \times 10^1$ ou plus et de $1,0 \times 10^5$ ou

moins.

**11.** Film de résine selon l'une quelconque des revendications 1 à 10, dans lequel le matériau de support de culture cellulaire ne contient pratiquement pas de matières premières d'origine animale.

**12.** Support de culture cellulaire, comportant :

un support ; et
le film de résine selon l'une quelconque des revendications 1 à 11,
le film de résine étant disposé sur une surface du support.

**13.** Récipient de culture cellulaire, comportant :

un corps de récipient ; et
le film de résine selon l'une quelconque des revendications 1 à 11,
le film de résine étant disposé sur une surface du corps de récipient.

[FIG. 1.]

[FIG. 2.]

Height Sensor

20.1 nm

1.0 µm

[FIG. 3.]

248.6 MPa

140.3 MPa

0.0          4: LogDMTModulus          1.0 µm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2006314285 A
- JP 2009524507 W

- JP 2017023008 A

### Non-patent literature cited in the description

- *Journal of the Adhesion Society of Japan*, 1986, vol. 22, 566
- *The Journal of Cell Biology*, September 1995, vol. 130 (5), 1189-1196
- *Protein, Nucleic Acid and Enzyme*, 2000, vol. 45 (15), 2477

- *Medicina Philosophica*, 1990, vol. 9 (7), 527-535
- *Journal of Osaka Women's and Children's Hospital*, 1992, vol. 8 (1), 58-66